(19) Europäisches Patentamt
European Patent Office
Office européen des brevets

(11) Publication number: **0 074 170**
**B1**

(12) **EUROPEAN PATENT SPECIFICATION**

(45) Date of publication of patent specification: **16.10.85**

(51) Int. Cl.[4]: **C 07 D 311/58,** C 07 D 311/64, C 07 D 409/06, A 61 K 31/35

(21) Application number: **82303965.6**

(22) Date of filing: **27.07.82**

(54) **Chroman compounds, process for producing them and pharmaceutical compositions containing them.**

(30) Priority: **12.08.81 JP 126912/81**

(43) Date of publication of application: **16.03.83 Bulletin 83/11**

(45) Publication of the grant of the patent: **16.10.85 Bulletin 85/42**

(84) Designated Contracting States: **AT BE CH DE FR GB IT LI LU NL SE**

(56) References cited:

**Chemical Abstracts vol. 68, no. 5, 29 January 1968, Columbus, Ohio, USA A.J. BIRCH et al. "The synthesis of o-isopentenylphenols", page 2089, column 2, abstract no. 21785d**

**Chemical Abstracts vol. 87, no. 9 29 August 1977, Columbus, Ohio, USA J. MAILLARD et al. "Antiinflammatory phenylacetic acid derivatives. III. 4-cyclohexylphenylacetic acid oxygenated derivatives", page 507, column 2, abstract no. 67940w**

(73) Proprietor: **NIPPON ZOKI PHARMACEUTICAL CO. LTD.
10, 2-chome Hirano-machi Higashi-ku
Osaka (JP)**

(72) Inventor: **Okamoto, Kaoru
10, 2-chome, Hiranomachi
Higashi-ku Osaka (JP)**
Inventor: **Hamada, Masaaki
10, 2-chome, Hiranomachi
Higashi-ku Osaka (JP)**
Inventor: **Kurosaki, Teikichi
10, 2-chome, Hiranomachi
Higashi-ku Osaka (JP)**

(74) Representative: **Crampton, Keith John Allen et al
D YOUNG & CO 10 Staple Inn
London WC1V 7RD (GB)**

**EP 0 074 170 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to heterocyclic compounds and their production, as well as to pharmaceutical compositions containing such compounds.

Although compounds such as salicylates, anthranilic acid derivatives, phenylacetic acid derivatives, indolylacetic acid derivatives and pyrazolones, have been used conventionally as drugs having antipyretic, analgesic and antiinflammatory actions, they have given rise to troubles due to side effects such as gastrointestinal, hepatorenal and haematological disorders. Allevation of these side effects has been intended by improving dosage forms and by chemical modification, or more recently by other methods including the use of appearance of pro-drugs.

Chem. Abs. *68* (1968), p. 2089, 21785d describes the preparation of an *o*-isopentenylphenol *via* an intermediate compound of formula

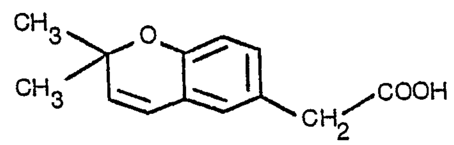

This intermediate is not described as exhibiting any pharmacological activity.

Chem. Abs. *87* (1977), p. 507, 67940w describes anti-inflammatory and analgesic α-phenylalkanoic acids of formula

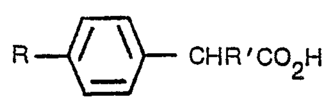

where R represents an oxocyclohexenyl, oxocyclohexyl, hydroxycyclohexenyl, hydroxycyclohexyl, oximinocyclohexenyl or oximinocyclohexyl group; and R′ represents a hydrogen atom or a methyl group.

The present invention results from investigations for developing drugs comparable or superior to conventional non-steroid drugs in effectiveness, and without the above-mentioned side effects. As a result, some types of new heterocyclic compounds were discovered, and certain of these compounds were found to have antiinflammatory and antipyretic-analgesic actions, as well as low toxicity.

The compounds included in this invention are represented by the following formula (I):

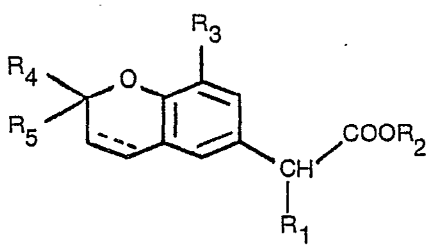

(I)

(in which each of $R_1$, $R_2$, $R_4$ and $R_5$ is a hydrogen atom or a $C_{1-6}$ alkyl group; $R_3$ is a hydrogen or halogen atom, a $C_{1-8}$ alkyl group optionally substituted by a $C_{3-6}$ cycloalkyl group or by one or more halogen atoms, a $C_{1-8}$ alkoxy group, a $C_{2-7}$ alkenyl group, a $C_{2-7}$ alkenyloxy group, a phenyl, tolyl, xylyl, biphenylyl, naphthyl, phenyloxy, tolyloxy, xylyloxy, biphenylyloxy or naphthyloxy group, an aliphatic, aromatic or heterocyclic acyl group, or a nitro group; and the broken line indicates that a single or double bond is present in the position shown; provided that when the broken line is a single bond, then $R_3$ is other than hydrogen, and provided that when the broken line is a double bond, $R_1$ is a hydrogen atom and $R_4$ and $R_5$ are both methyl groups, then $R_3$ is other than hydrogen) and compounds that are pharmaceutically acceptable salts thereof.

Values of $R_1$, $R_2$, $R_4$ and $R_5$ include straight and branched $C_{1-6}$, e.g. $C_{1-5}$ and preferably $C_{1-4}$, alkyl groups, e.g. methyl, ethyl, propyl, *i*-propyl, butyl, *i*-butyl, sec-butyl and *t*-butyl; values of $R_3$, apart from hydrogen and nitro, include fluorine, chlorine, bromine, iodine; straight or branched $C_{1-8}$ alkyl groups, e.g. methyl, ethyl, propyl, *i*-propyl, butyl, *i*-butyl, *sec*-butyl and *t*-butyl as well as pentyl, hexyl, heptyl and octyl, including their branched isomers, and $C_{1-8}$ alkyl substituted with a $C_{3-6}$ cycloalkyl group, e.g. cyclopropylmethyl, cyclobutylmethyl, cyclopentylmethyl and cyclohexylmethyl; the above-mentioned alkyl groups substituted with one or more halogen atoms, e.g. chloromethyl, chloroethyl, fluoromethyl, fluoroethyl, bromomethyl, bromoethyl and trifluoromethyl; alkoxy groups corresponding to the above-mentioned alkyl groups; straight or branched alkenyl groups with 2 to 7 carbon atoms such as vinyl, propenyl, butenyl, pentenyl, hexenyl and heptenyl and especially methallyl and allyl; alkenyloxy groups corresponding to these alkenyl groups; aryl groups selected from phenyl, tolyl, xylyl, biphenylyl and naphthyl; aryloxy groups corresponding to these aryl groups; and acyl groups selected from aliphatic acyl groups such as

acetyl, propionyl, butyryl and valeryl, aromatic acyl groups such as benzoyl, toluoyl, naphthoyl and cinnamoyl, and heterocyclic acyl groups such as furoyl and thenoyl.

When optical isomers exist in the above-mentioned compounds, the present invention includes any of the *dl*-, *l*- and *d*-isomers.

This invention includes the following specific compounds:
(8-chloro-1,2-benzopyran-6-yl)acetic acid,
2-(8-chlorochroman-6-yl)propionic acid,
2-(2,2-dimethyl-8-chlorochroman-6-yl)propionic acid,
2-(8-chloro-1,2-benzopyran-6-yl)propionic acid,
2-(2,2-dimethyl-8-chloro-1,2-benzopyran-6-yl)propionic acid,
2-(2,2-dimethyl-1,2-benzopyran-6-yl)propionic acid,
2-(2,2-dimethyl-8-fluoro-1,2-benzopyran-6-yl)propionic acid,
2-(2,2-dimethyl-8-phenyl-1,2-benzopyran-6-yl)propionic acid,
2-(8-phenyl-1,2-benzopyran-6-yl)propionic acid,
2-(2,2-dimethyl-8-benzoyl-1,2-benzopyran-6-yl)propionic acid,
2-(8-isopropyl-1,2-benzopyran-6-yl)propionic acid,
(2,2-dimethyl-8-methoxy-1,2-benzopyran-6-yl)acetic acid,
2-(2,2-dimethyl-8-thenoyl-1,2-benzopyran-6-yl)propionic acid,
(2,2-dimethyl-8-nitro-1,2-benzopyran-6-yl)acetic acid,
(2,2-dimethyl-8-phenoxy-1,2-benzopyran-6-yl)acetic acid,
as well as lower alkyl esters of these compounds.

The present invention also includes pharmaceutically acceptable salts of the compounds, and those with alkali metals such as sodium and potassium, with alkaline-earth metals such as calcium and magnesium, and with ammonium, are especially preferred when the compounds are free carboxylic acids.

In a further aspect, the invention provides pharmaceutical compositions comprising as active ingredient at least one compound according to the invention together with a pharmaceutical diluent, carrier or coating.

In accordance with the present invention the new compounds are prepared as follows. A compound represented by the general formula (II):

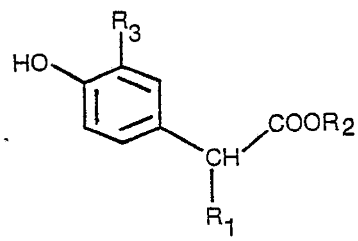

(II)

where $R_1$, $R_2$ and $R_3$ have the same meanings as those in the above-mentioned general formula (I), is reacted with a compound represented by the general formula (III):

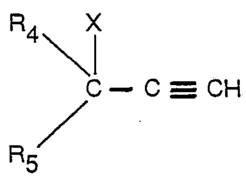

(III)

where $R_4$ and $R_5$ have the same meanings as those in the above-mentioned general formula (I), and X indicates a halogen, to give a compound represented by a general formula (IV):

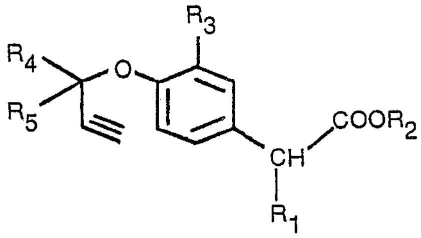

(IV)

where $R_1$, $R_2$, $R_3$, $R_4$ and $R_5$ have the same meanings as those in the above-mentioned general formula (I), and the compound (IV) is converted to a 1,2-benzopyran compound of general formula (I) by heating and ring closure.

To conveniently carry out the reaction, the compounds represented by the general formulae (II) and (III)

are allowed to stand at room temperature or are heated at an appropriate temperature in an inert solvent such as benzene, toluene, xylene, acetone, methyl ethyl ketone, diethyl ether, methyl isobutyl ketone, tetrahydrofuran, dioxane, dimethoxyethane, dimethylformamide or dimethyl sulphoxide, preferably in acetone or dimethylformamide, in the presence of a base such as sodium carbonate, potassium carbonate, calcium carbonate, sodium bicarbonate, sodium hydroxide, potassium hydroxide, or a tertiary amine, with potassium carbonate being preferred; if necessary, reaction promoters such as sodium iodide and potassium iodide are added.

The resulting compound (IV) is then heated generally in a solvent such as dimethylaniline or diethylaniline at up to 190—220°C to yield by ring closure nearly quantitative amounts of the intended compounds.

Further, reduction of the obtained 1,2-benzopyran compounds in the usual way, for example, in the presence of catalysts such as platinum oxide, palladium charcoal or Raney nickel gives rise to the chroman compounds of the present invention.

The reaction of alkyne derivatives corresponding to the above-mentioned compounds (III) with the above-mentioned compounds (II) followed by treatment with acids such as sulphuric acid or boron trifluoride etherate may produce the chroman compounds of the present invention.

The hydrolysis of esters to obtain the corresponding free carboxylic acids of the present invention can be conducted in appropriate solvents such as water, ethanol, methanol, or mixtures of them, using an alkali metal hydroxide, preferably sodium hydroxide or potassium hydroxide, at room temperature or under reflux for 30 minutes to 2 hours.

The compounds of this invention also may be converted to their salts, if desired, in the usual way.

The compounds of this invention can be purified by usual methods such as distillation, chromatography and recrystallization. They are identifiable by, *inter alia*, elementary analysis, melting point, IR, NMR, UV and mass spectrum.

The examples, which follow, describe the preparation of the compounds of the present invention. Certain of the experimental results require reference to the accompanying drawings, in which:

Figure 1 illustrates antiinflammatory effects of the compounds of the present invention, and

Figure 2 illustrates antipyretic effects of the compounds of the present invention.

## Example 1

To 60 ml of dimethylformamide were added 8.5 g of methyl 2-(3-fluoro-4-hydroxyphenyl)propionate, 10.7 g of potassium iodide and 11.9 g of potassium carbonate. 6.3 ml of 3-chloro-3-methyl-1-butyne was further added dropwise and the mixture was reacted at 75°C for 18 hours, followed by addition of water and extraction with ether. The ethereal solution was washed with water and a saturated solution of sodium chloride and dried over anhydrous sodium sulphate, whereupon the solvent was distilled off. The residue was purified by column chromatography on silica gel (benzene) to obtain 9.1 g of methyl 2-[3-fluoro-4-(1,1-dimethylpropargyloxy)-phenyl]propionate. To 9 g of the latter was added 45 ml of dimethylaniline and the mixture was heated under reflux at 210°C for 3 hours; after cooling, ether was added and the mixture was washed with 5% hydrochloric acid, water and saturated sodium chloride. Then the same treatment was carried out as described above to give 7.3 g of methyl 2-(2,2-dimethyl-8-fluoro-1,2-benzopyran-6-yl)propionate.

70 ml of methanol and 7 ml of 50% potassium hydroxide were added to the compound obtained and the mixture refluxed for 1 hour; methanol was distilled off under reduced pressure and water was added to the residue, which was washed with ether. Concentrated hydrochloric acid was added to the aqueous layer, which then was extracted with ether. The ethereal layer was washed with water and saturated sodium chloride and dried over anhydrous sodium sulphate, and the crystals obtained were recrystallized from a mixture of *n*-hexane and ethyl acetate to give quantitatively 2-(2,2-dimethyl-8-fluoro-1,2-benzopyran-6-yl)propionic acid (compound 1).

mp: 96—98°C

IR (KBr): 3400—2100, 2970, 1700, 1635, 1619, 1582, 1488, 1379, 1272, 1211, 1142, 1055, 905, 722, 669 $cm^{-1}$

NMR ($CDCl_3$): $\delta$ = 1.46 (6H, s), 1.46 (3H, d, J = 7 Hz), 3.60 (1H, q, J = 7 Hz), 5.62 (1H, d, J = 10 Hz), 6.28 (1H, dd, $J_1$ = 10 Hz, $J_2$ = 2 Hz), 6.6—7.0 (2H, m), 11.07 (1H, br.s)

## Example 2

To 100 ml of dimethylformamide were added 10.0 g of ethyl 3-methoxy-4-hydroxyphenylacetate, 11.8 g of potassium iodide and 13.1 g of potassium carbonate, and then 8.1 ml of 3-chloro-3-methyl-1-butyne was added dropwise and the mixture was stirred at 70 to 80°C for 20 hours. After cooling, water was added to the mixture, which was extracted with ether; the ether layer was washed with water, 10% sodium hydroxide and saturated solution of sodium chloride, and dried over anhydrous sodium sulfate; the solvent was distilled off; and the residue was purified by column chromatography on silica gel (benzene/ethyl acetate = 10/1) to give 6.0 g of ethyl 3-methoxy-4-(1,1-dimethylpropargyloxy)pheny]acetate. The obtained compound was dissolved in 60 ml of dimethylaniline and refluxed at 210 to 220°C for 4 hours, and then ether was added to the reaction mixture and, in the same way as in the case of example 1, 5.7 g of ethyl (2,2-dimethyl-8-methoxy-1,2-benzopyran-6-yl)acetate was obtained.

6 ml of 50% potassium hydroxide was added to a solution of 5.7 g of the above compound in 60 ml of ethanol and the mixture was stirred at 70°C for 30 minutes, then, after ethanol was distilled off, water was added to the resultant residue, which was washed with ether; and the aqueous layer was made acidic by hydrochloric acid and the crystals which separated out were recrystallized from a mixture of n-hexane and ethyl acetate to give quantitatively (2,2-dimethyl-8-methoxy-1,2-benzopyran-6-yl)acetic acid.

mp: 109—111°C

IR (KBr): 3500—2200, 2955, 1690, 1630, 1581, 1485, 1376, 1209, 1148, 1090, 950, 720, 640 $cm^{-1}$

NMR (Acetone-$D_6$): δ = 1.39 (6H, s), 3.49 (2H, s), 3.78 (3H, s), 5.66 (1H, d, J = 10 Hz), 6.31 (1H, d, J = 10 Hz), 6.59 (1H, d, J = 2 Hz), 6.79 (1H, d, J = 2 Hz), 7.8—9.5 (1H, br.)

In the same way, the following compounds were obtained.

Methyl 2-(2,2-dimethyl-8-benzoyl-1,2-benzopyran-6-yl)propionate,
Methyl 2-(2,2-dimethyl-8-phenyl-1,2-benzopyran-6-yl)propionate,
Methyl 2-(2,2-dimethyl-1,2-benzopyran-6-yl)propionate,
Methyl 2-(2,2-dimethyl-8-chloro-1,2-benzopyran-6-yl)propionate,
Methyl 2-(2,2-dimethyl-8-thenoyl-1,2-benzopyran-6-yl)propionate,
Methyl 2-(8-phenyl-1,2-benzopyran-6-yl)propionate,
Methyl 2-(8-isopropyl-1,2-benzopyran-6-yl)propionate,
Methyl 2-(8-chloro-1,2-benzopyran-6-yl)propionate,
Methyl (2,2-dimethyl-8-nitro-1,2-benzopyran-6-yl)acetate,
Methyl (2,2-dimethyl-8-phenoxy-1,2-benzopyran-6-yl)acetate,
Methyl (8-chloro-1,2-benzopyran-6-yl)acetate,

2-(2,2-Dimethyl-8-benzoyl-1,2-benzopyran-6-yl)propionic acid;

mp: 161—163°C

IR (KBr): 3400—2000, 2960, 1700, 1650, 1584, 1576, 1450, 1205, 907, 738, 715, 675 $cm^{-1}$

NMR ($CDCl_3$): δ = 1.19 (6H, s), 1.49 (3H, d, J = 7 Hz), 3.65 (1H, q, J = 7 Hz), 5.53 (1H, d, J = 10 Hz), 6.39 (1H, d, J = 10 Hz), 7.02 (1H, d, J = 2 Hz), 7.15 (1H, d, J = 2 Hz), 7.2—8.0 (5H, m), 10.77 (1H, br.s)

2-(2,2-Dimethyl-8-phenyl-1,2-benzopyran-6-yl)propionic acid;

mp: 129—131°C

IR (KBr): 3400—2000, 2970, 1698, 1596, 1499, 1460, 1258, 1132, 940, 759, 695 $cm^{-1}$

NMR ($CDCl_3$): δ = 1.39 (6H, s), 1.50 (3H, d, J = 7 Hz), 3.65 (1H, q, J = 7 Hz), 5.59 (1H, d, J = 10 Hz), 6.41 (1H, d, J = 10 Hz), 6.90 (1H, d, J = 2 Hz), 7.10 (1H, d, J = 2 Hz), 7.15—7.7 (5H, m), 9.57 (1H, br.s)

2-(2,2-Dimethyl-1,2-benzopyran-6-yl)propionic acid (compound 2);

mp: 82—84°C

IR (KBr): 3600—2100, 2970, 1695, 1632, 1600, 1486, 1260, 1200, 1145, 955, 765, 715 $cm^{-1}$

NMR ($CDCl_3$): δ = 1.38 (6H, s), 1.45 (3H, d, J = 7 Hz), 3.60 (1H, q, J = 7 Hz), 5.51 (1H, d, J = 10 Hz), 6.24 (1H, d, J = 10 Hz), 6.6—7.2 (3H, m), 11.53 (1H, s)

2-(2,2-Dimethyl-8-chloro-1,2-benzopyran-6-yl)propionic acid (compound 3);

mp: 88—90°C

IR (KBr): 3600—2200, 2970, 1705, 1630, 1562, 1465, 1270, 1205, 1140, 940, 900, 765, 720 $cm^{-1}$

NMR ($CDCl_3$): δ = 1.46 (6H, s), 1.47 (3H, d, J = 7 Hz), 3.59 (1H, q, J = 7 Hz), 5.61 (1H, d, J = 10 Hz), 6.26 (1H, d, J = 10 Hz), 6.80 (1H, d, J = 2 Hz), 7.11 (1H, d, J = 2 Hz), 11.15 (1H, br.s)

2-(2,2-Dimethyl-8-thenoyl-1,2-benzopyran-6-yl)propionic acid;

mp: 133—135°C

IR (KBr): 3400—2200, 2970, 1700, 1633, 1580, 1519, 1450, 1411, 1268, 1208, 1145, 903, 760, 721, 678 $cm^{-1}$

NMR (Acetone-$D_6$): δ = 1.30 (6H, s), 1.46 (3H, d, J = 7 Hz), 3.75 (1H, q, J = 7 Hz), 5.73 (1H, d, J = 10 Hz), 6.45 (1H, d, J = 10 Hz), 7.0—7.3 (3H, m), 7.49 (1H, dd, $J_1$ = 4 Hz, $J_2$ = 1.5 Hz), 7.78 (1H, dd, $J_1$ = 5 Hz, $J_2$ = 1.5 Hz), 9.07 (1H, br.s)

2-(8-Phenyl-1,2-benzopyran-6-yl)propionic acid;

mp: 143—145°C

IR (KBr): 3400—2000, 1709, 1600, 1500, 1472, 1215, 1130, 1032, 895, 764, 701 $cm^{-1}$

NMR ($CDCl_3$): δ = 1.49 (3H, d, J = 7 Hz), 3.67 (1H, q, J = 7 Hz), 4.75 (2H, dd, $J_1$ = 3 Hz, $J_2$ = 1.5 Hz), 5.77 (1H, dt, $J_1$ = 10 Hz, $J_2$ = 3 Hz), 6.43 (1H, dt, $J_1$ = 10 Hz, $J_2$ = 1.5 Hz), 6.90 (1H, d, J = 2 Hz), 7.10 (1H, d, J = 2 Hz), 7.15—7.7 (5H, m), 9.47 (1H, br.s)

2-(8-Isopropyl-1,2-benzopyran-6-yl)propionic acid;

mp: 78—80°C

IR (KBr): 3500—2000, 2950, 1700, 1582, 1467, 1204, 1142, 920, 705 $cm^{-1}$

NMR ($CDCl_3$): δ = 1.19 (6H, d, J = 7 Hz), 1.47 (3H, d, J = 7 Hz), 3.20 (1H, sep, J = 7 Hz), 3.61 (1H, q,

J = 7 Hz), 4.74 (2H, dd, $J_1$ = 3.5 Hz, $J_2$ = 1.5 Hz), 5.73 (1H, dt, $J_1$ = 10 Hz, $J_2$ = 3.5 Hz), 6.37 (1H, dt, $J_1$ = 10 Hz, $J_2$ = 1.5 Hz), 6.74 (1H, d, J = 2 Hz), 6.94 (1H, d, J = 2 Hz), 10.97 (1H, br.s)

2-(8-Chloro-1,2-benzopyran-6-yl)propionic acid (compound 4);
mp: 97—99°C
IR (KBr): 3500—2100, 1700, 1635, 1560, 1472, 1324, 1220, 1138, 1029, 920, 885, 720, 685 $cm^{-1}$
NMR ($CDCl_3$): δ = 1.44 (3H, d, J = 7 Hz), 3.58 (1H, q, J = 7 Hz), 4.88 (2H, dd, $J_1$ = 4 Hz, $J_2$ = 2 Hz), 5.74 (1H, dt, $J_1$ = 10 Hz, $J_2$ = 4 Hz), 6.34 (1H, dt, $J_1$ = 10 Hz, $J_2$ = 2 Hz), 6.77 (1H, d, J = 2 Hz), 7.09 (1H, d, J = 2 Hz), 11.43 (1H, br.s)

(2,2-Dimethyl-8-nitro-1,2-benzopyran-6-yl)acetic acid;
mp: 149—151°C
IR (KBr): 3600—2200, 1705, 1635, 1610, 1568, 1520, 1265, 1230, 1140, 948, 750, 721 $cm^{-1}$
NMR (Acetone-$D_6$): δ = 1.48 (6H, s), 3.63 (2H, s), 5.88 (1H, d, J = 10 Hz), 6.48 (1H, d, J = 10 Hz), 7.24 (1H, d, J = 2 Hz), 7.60 (1H, d, J = 2 Hz), 9.5—10.5 (1H, br)

(2,2-Dimethyl-8-phenoxy-1,2-benzopyran-6-yl)acetic acid;
mp: 148—150°C
IR (KBr): 3500—2100, 1700, 1621, 1584, 1560, 1490, 1360, 1202, 1151, 835, 758, 692 $cm^{-1}$
NMR (Acetone-$D_6$): δ = 1.26 (6H, s), 3.75 (2H, s), 5.84 (1H, d, J = 10 Hz), 6.69 (1H, d, J = 10 Hz), 6.8—7.5 (7H, m), 10.0—11.0 (1H, br)

(8-Chloro-1,2-benzopyran-6-yl)acetic acid (compound 5);
mp: 137—139°C
IR (KBr): 3400—2000, 1690, 1637, 1599, 1562, 1478, 1301, 1225, 1217, 1136, 911, 860, 721, 689, 631 $cm^{-1}$
NMR (Acetone-$D_6$): δ = 3.52 (2H, s), 4.87 (2H, dd, $J_1$ = 4 Hz, $J_2$ = 1.5 Hz), 5.83 (1H, dt, $J_1$ = 10 Hz, $J_2$ = 4 Hz), 6.40 (1H, dt, $J_1$ = 10 Hz, $J_2$ = 1.5 Hz), 6.84 (1H, d, J = 2 Hz), 7.10 (1H, d, J = 2 Hz), 8.34 (1H, br.s)

Example 3

10 ml of methanol and 10 mg of platinum oxide were added to 1 g of methyl 2-(2,2-dimethyl-8-chloro-1,2-benzopyran-6-yl)propionate and vigorously stirred in a stream of hydrogen for 15 minutes and then filtered, and the filtrate was distilled off to give methyl 2-(2,2-dimethyl-8-chlorochroman-6-yl)propionate. 10 ml of methanol and 1 ml of 50% potassium hydroxide were added to the compound and refluxed for 1 hour; methanol was distilled off under reduced pressure; and the crystals, obtained after adding water and acidifying by hydrochloric acid, were recrystallized from a mixture of n-hexane and ether to give quantitatively white crystals of 2-(2,2-dimethyl-8-chlorochroman-6-yl)propionic acid (compound 6).
mp: 116—118°C
IR (KBr): 3500—2100, 2970, 1702, 1600, 1568, 1475, 1231, 1146, 1115, 920, 767, 718, 668 $cm^{-1}$
NMR ($CDCl_3$): δ = 1.34 (6H, s), 1.44 (3H, d, J = 7 Hz), 1.77 (2H, t, J = 7 Hz), 2.74 (2H, t, J = 7 Hz), 3.58 (1H, q, J = 7 Hz), 6.87 (1H, d, J = 2 Hz), 7.11 (1H, d, J = 2 Hz), 11.15 (1H, br.s)
The following compounds were obtained in the same way.

Methyl 2-(8-chlorochroman-6-yl)propionate, 2-(8-Chlorochroman-6-yl)propionic acid;
mp: 135—137°C
IR (KBr): 3500—2000, 1700, 1565, 1480, 1422, 1322, 1230, 1130, 1058, 940, 865, 720 $cm^{-1}$
NMR ($CDCl_3$): δ = 1.45 (3H, d, J = 7 Hz), 1.98 (2H, tt, $J_1$ = 6 Hz, $J_2$ = 5 Hz), 2.77 (2H, t, J = 6 Hz), 3.58 (1H, q, J = 7 Hz), 4.24 (2H, t, J = 5 Hz), 6.83 (1H, d, J = 2 Hz), 7.09 (1H, d, J = 2 Hz), 10.98 (1H, br.s)

Compounds of the present invention have new structures different from conventional non-steroid compounds and remarkably potent antiinflammatory, analgesic and antipyretic actions as well as low toxicity.

The following descriptions serve to illustrate animal studies.

(1) Acute toxicity

Groups of 10 male dd mice weighing some 18 g were orally administered 800 mg/Kg of the examined drugs and the number of deaths up to 72 hours later was recorded.

An example of results is shown in Table 1.

TABLE 1

| Drugs | Mortality (%) |
|---|---|
| Aminopyrine | 80 |
| Phenylbutazone | 60 |
| Compound 1 | 0 |
| Compound 2 | 0 |
| Compound 3 | 0 |
| Compound 4 | 30 |
| Compound 6 | 30 |
| Compound 7 | 0 |

(2) Inhibition of plantar oedema induced by carrageenin

Sixty minutes after the drugs had been orally administered to groups of 5 Wistar rats, 0.1 ml of 1% carrageenin solution was injected subcutaneously into the sole. The volume of the hind foot was determined thereafter over 5 hours at intervals of 1 hour. The rate of increase in volume compared with the volume before carrageenin treatment was regarded as oedema rate.

An example of the results is shown in Fig. 1.

Furthermore, dissection of the stomach and intestinal tracts of the rats after the above-mentioned observation is over revealed mucosal disorder or formation of ulcer in the phenylbutazone-administered group, while no abnormality was found in any animals in the group which received the compounds of the present invention.

(3) Analgesic effects by a modified Haffner method

Groups of 10 dd mice were orally administered the examined drugs and, 30 minutes later, 2 mg/Kg of morphine hydrochloride, the threshold dose, was injected subcutaneously. At 15, 30, 45 and 60 minutes thereafter, the root of mouse's tail was squeezed with a Kocher's forceps. The number of animals that did not exhibit pseudopain reactions was recorded and the determination with the largest number of responding animals among the four determinations was used for evaluation.

An example of results is shown in Table 2.

TABLE 2

| Drugs | Analgesic effects (%) 50 mg/Kg | Analgesic effects (%) 100 mg/Kg |
|---|---|---|
| Aminopyrine | 20 | 55 |
| Compound 1 | 40 | 70 |
| Compound 2 | 25 | 50 |
| Compound 3 | 20 | 65 |
| Compound 4 | 30 | 55 |
| Compound 5 | 35 | 70 |
| Compound 6 | 30 | 60 |

(4) Analgesic effects by acetic acid writhing test

Thirty minutes after the examined drug was orally given, 0.1 ml/10 g of 0.6% acetic acid was intraperitoneally administered to groups of 10 male dd mice weighing some 18 g. The number of stretching was

calculated during an observation period of 15 to 20 minutes after acetic acid treatment. The average inhibition rate of stretching among 8 animals was regarded as the inhibition rate of the drug, excluding 2 animals showing maximum and minimum numbers of stretching.

An example of results is shown in Table 3.

TABLE 3

| Drugs | Analgesic effects (%) 50 mg/Kg | 100 mg/Kg |
|---|---|---|
| Aminopyrine | 18 | 57 |
| Compound 1 | 59 | 76 |
| Compound 2 | 44 | 76 |
| Compound 4 | 36 | 61 |
| Compound 6 | 27 | 68 |

(5) Antipyretic effects

After the normal rectal temperature was measured, 400 mg/Kg of beer yeast was injected subcutaneously to female Wistar rats weighing 165—185 g. The body temperature of the animals was measured at intervals of one hour after the yeast treatment. The animals which exhibited appreciable rise in the body temperature were selected to make groups of 10 animals. The examined drug was orally administered to the selected animals 4 hours after the yeast treatment and during 4 hours thereafter measurements of the temperature were made at intervals of one hour to examine antipyretic effect.

As shown in Fig. 2, the compounds of the present invention indicated excellent antipyretic activity.

As the above-mentioned animal experiments clearly indicate, the compounds of the present invention have excellent antiinflammatory, analgesic and antipyretic actions. Therefore, these compounds are therapeutically available antiinflammatory, analgesic and antipyretic agents against various inflammations such as those in rheumatic diseases, arthritis, and other inflammations having a variety of redness, fever, swelling and pain, against painful diseases and manifestations such as acute and chronic pains, neuralgia, pains accompanied by inflammation, trauma and lumbago, and against a variety of symptoms having fever.

The compounds of the present invention can be made into pharmaceuticals by combination with appropriate medicinal carriers or diluents, and dosage forms of solids, semisolids, liquids or gases can be prepared in a usual way for oral or non-oral administration.

On preparation of pharmaceutical dosage forms, the compounds of the present invention may be used in the form of their pharmaceutically acceptable salts, and they also can be used alone or in appropriate association thereof, as well as in combination with other pharmaceutically active components.

When the compounds are applied orally, they may be used alone or combined with appropriate fillers to make tablets or capsules, e.g. with conventional bases such as lactose, mannitol, corn starch, potato starch, with binders such as crystalline cellulose, cellulose derivatives, acacia, corn starch or gelatin, with disintegrators such as corn starch, potato starch or sodium carboxymethylcellulose, and with lubricants such as talc and magnesium stearate. They also can be combined with ointment bases such as vaseline, paraffin, plastibase, simple ointment, hydrophilic ointment, hydrophilic petrolatum and hydrophilic plastibase to make ointments. Further, the compounds of the present invention may be mixed thoroughly with a variety of bases such as emulsifying bases or water-soluble bases to give suppositories.

As regards injectable forms, these compounds can be administered subcutaneously, intramuscularly or intravenously as solutions or suspensions in aqueous solvents or non-aqueous solvents such as vegetable oils, synthetic aliphatic acid glycerides, esters of higher aliphatic acids and propylene glycol. When utilized as inhalation or aerosol preparations, the compounds of the present invention in the form of a liquid or minute powder can be filled up in an aerosol container, with gas or liquid spraying agents, and with conventional adjuvants such as humidifying or dispersing agents added, if necessary. The compounds of the present invention also may be applied as pharmaceuticals for a neblizer or an atomizer.

Poultices can be prepared by mixing the compounds with mentha oil, concentrated glycerin, kaolin, or other suitable additives.

Liniments can be prepared by adding oils, e.g. fatty oils or essential oils, and if necessary, emulsifying agents such as stearic acid and oleic acid.

The desirable dose of the compounds of the present invention varies with the subject, method and period of administration, but generally it is recommended to administer orally 10 to 3,000 mg of these

compounds daily to an adult to obtain the desired effects. One to several units of the unit preparation containing the compound of the present invention in appropriate amount may be administered.

As for non-oral administration (e.g. for injectional forms), doses in the order of one tenth to one third of the above oral dose are desirable as daily doses.

Some prescriptions of the pharmaceutical compositions are shown below as examples which contain the compounds of the present invention as active ingredients.

Prescription example 1. (tablet)

| Components | Content of a tablet (mg) |
|---|---|
| an invented compound | 50 |
| lactose | 130 |
| corn starch | 60 |
| magnesium stearate | 10 |
| | total 250 mg |

Prescription example 2. (capsule)

| Components | Content of a capsule (mg) |
|---|---|
| an invented compound | 100 |
| lactose | 200 |
| | total 300 mg |

Prescription example 3. (injection)

| Components | Content of an ampoule (mg) |
|---|---|
| an invented compound | 10 |
| sodium chloride | proper amount |
| distilled water for injection | proper amount |
| | total 1 ml |

Prescription example 4. (ointment)

| Components | Weight (g) |
|---|---|
| an invented compound | 1 |
| emulsified wax | 30 |
| white petrolatum | 50 |
| liquid paraffin | 20 |
| | total 101 g |

Prescription example 5. (suppository)

| Components | Content of a suppository (mg) |
| --- | --- |
| an invented compound | 20 |
| cacao butter | 1980 mg |
| | total 2000 mg |

Prescription example 6. (aerosol)

| Components | Weight (%) |
| --- | --- |
| an invented compound | 1 |
| isopropyl myristate | 1 |
| dichlorodifluoromethane | 39 |
| dichlorotetrafluoroethane | 59 |
| | total 100% |

Prescription example 7. (poultice)

| Components | Content |
| --- | --- |
| an invented compound | 10 g |
| conc. glycerin | 450 g |
| mentha oil | 0.5 ml |
| kaolin | 540 g |
| | total 1000 g |

Prescription example 8. (liniment)

| Components | Content |
| --- | --- |
| an invented compound | 10 g |
| potash soap | 80 g |
| camphor | 20 g |
| thyme oil | 4 ml |
| mentha oil | 6 ml |
| ammonia water | 50 ml |
| ethanol | 840 ml |
| purified water | proper amount |
| | total 1000 ml |

**Claims for the Contracting States: BE CH DE FR GB IT LI LU NL SE**

1. Heterocyclic compounds of the general formula (I):

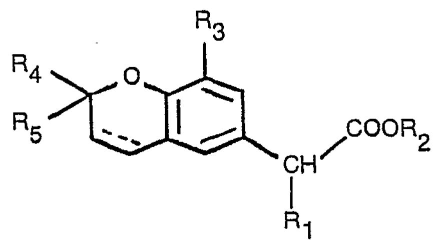

(I)

(in which each of $R_1$, $R_2$, $R_4$ and $R_5$ is a hydrogen atom or a $C_{1-6}$ alkyl group; $R_3$ is a hydrogen or halogen atom, a $C_{1-8}$ alkyl group optionally substituted by a $C_{3-6}$ cycloalkyl group or by one or more halogen atoms, a $C_{1-8}$ alkoxy group, a $C_{2-7}$ alkenyl group, a $C_{2-7}$ alkenyloxy group, a phenyl, tolyl, xylyl, biphenylyl, naphthyl, phenyloxy, tolyloxy, xylyloxy, biphenylyloxy or naphthyloxy group, an aliphatic, aromatic or heterocyclic acyl group, or a nitro group; and the broken line indicates that a single or double bond is present in the position shown; provided that when the broken line is a single bond, then $R_3$ is other than hydrogen, and provided that when the broken line is a double bond, $R_1$ is a hydrogen atom and $R_4$ and $R_5$ are both methyl groups, then $R_3$ is other than hydrogen) and pharmaceutically acceptable salts thereof.

2. 2-(2,2-Dimethyl-8-fluoro-1,2-benzopyran-6-yl)propionic acid.

3. (2,2-Dimethyl-8-methoxy-1,2-benzopyran-6-yl)acetic acid.

4. 2-(2,2-Dimethyl-8-chlorochroman-6-yl)propionic acid.

5. A method of preparing a compound as claimed in claim 1, comprising reacting a compound of the general formula (II):

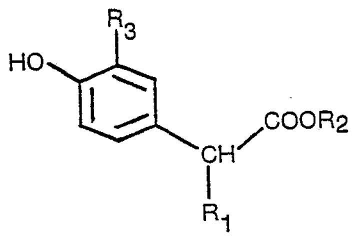

(II)

where $R_1$, $R_2$ and $R_3$ are as defined in claim 1 with a compound of the general formula (III):

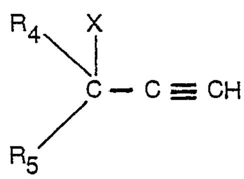

(III)

where $R_4$ and $R_5$ are as defined in claim 1 and X is a halogen atom, to give a compound of the general formula (IV):

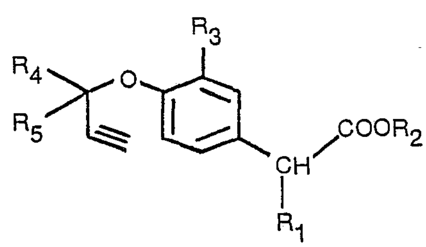

(IV)

where $R_1$, $R_2$, $R_3$, $R_4$ and $R_5$ are as defined in claim 1, heating the latter to effect ring closure, and, if desired, reducing, hydrolysing or converting to a salt the resulting ring-closed compound.

6. A process according to claim 5 wherein the compound of formula (II) is reacted with the compound of formula (III) in the presence of a base.

7. A process according to either of claims 5 and 6 wherein the ring closure is effected by heating the compound of formula (IV) in a solvent.

8. A pharmaceutical composition comprising as active ingredient at least one compound as claimed in any one of claims 1 to 4, together with a pharmaceutical diluent, carrier or coating.

9. For use in the treatment of inflammation, fever or pain, a compound as claimed in any one of claims 1 to 4.

**Claims for the Contracting State: AT**

1. A process for the preparation of heterocyclic compounds of the general formula (I):

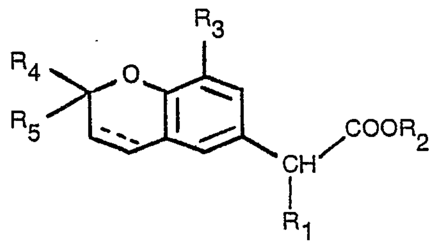

(I)

(in which each of $R_1$, $R_2$, $R_4$ and $R_5$ is a hydrogen atom or a $C_{1-6}$ alkyl group; $R_3$ is a hydrogen or halogen atom, a $C_{1-8}$ alkyl group optionally substituted by a $C_{3-6}$ cycloalkyl group or by one or more halogen atoms, a $C_{1-8}$ alkoxy group, a $C_{2-7}$ alkenyl group, a $C_{2-7}$ alkenyloxy group, a phenyl, tolyl, xylyl, biphenylyl, naphthyl, phenyloxy, tolyloxy, xylyloxy, biphenylyloxy or naphthyloxy group, an aliphatic, aromatic or heterocyclic acyl group, or a nitro group; and the broken line indicates that a single or double bond is present in the position shown; provided that when the broken line is a single bond, then $R_3$ is other than hydrogen, and provided that when the broken line is a double bond, $R_1$ is a hydrogen atom and $R_4$ and $R_5$ are both methyl groups, then $R_3$ is other than hydrogen) and pharmaceutically acceptable salts thereof, which comprises reacting a compound of the general formula (II)

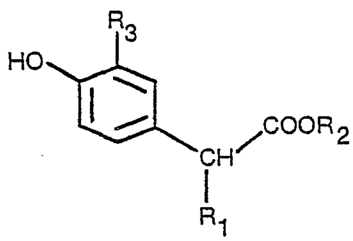

(II)

where $R_1$, $R_2$ and $R_3$ are as defined in claim 1 with a compound of the general formula (III):

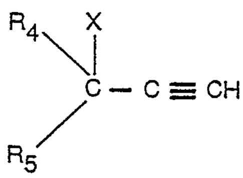

(III)

where $R_4$ and $R_5$ are as defined in claim 1 and X is a halogen atom, to give a compound of the general formula (IV):

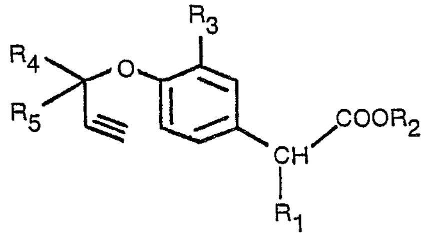

(IV)

where $R_1$, $R_2$, $R_3$, $R_4$ and $R_5$ are as defined in claim 1, heating the latter to effect ring closure, and, if desired, reducing, hydrolysing or converting to a salt the resulting ring-closed compound.

2. A process according to claim 1 wherein the compound of formula (I) is 2-(2,2-dimethyl-8-fluoro-1,2-benzopyran-6-yl)propionic acid.

3. A process according to claim 1 wherein the compound of formula (I) is (2,2-dimethyl-8-methoxy-1,2-benzopyran-6-yl)acetic acid.

4. A process according to claim 1 wherein the compound of formula (I) is 2-(2,2-dimethyl-8-chlorochroman-6-yl)propionic acid.

5. A process according to any one of the preceding claims wherein the compound of formula (II) is reacted with the compound of formula (III) in the presence of a base.

6. A process according to any one of the preceding claims wherein the ring closure is effected by heating the compound of formula (IV) in a solvent.

7. A process for the preparation of a pharmaceutical composition which comprises combining a compound of formula (I) as defined in any one of claims 1 to 4 or a pharmaceutically acceptable salt thereof with a pharmaceutical diluent, carrier or coating.

**Patentansprüche für die Vertragsstaaten: BE CH DE FR GB IT LI LU NL SE**

1. Heterozyklische Verbindungen der allgemeinen Formel (I)

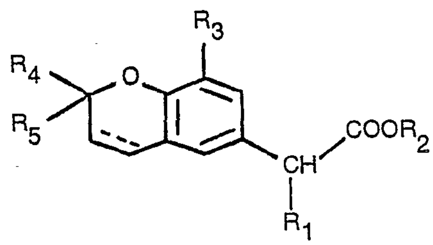

(I)

(worin jeweils $R_1$, $R_2$, $R_4$ und $R_5$ ein Wasserstoffatom oder eine $C_{1-6}$-Alkylgruppe bedeutet, $R_3$ ein Wasserstoffatom oder Halogenatom, eine $C_{1-8}$-Alkylgruppe, die gegebenenfalls durch eine $C_{3-6}$-Cycloalkylgruppe oder durch ein oder mehrere Halogenatome substituiert ist, eine $C_{1-8}$-Alkoxygruppe, eine $C_{2-7}$-Alkenylgruppe, eine $C_{2-7}$-Alkenyloxygruppe, eine Phenyl-, Tolyl-, Xylyl-, Biphenylyl-, Naphthyl-, Phenyloxy-, Tolyloxy-, Xylyloxy-, Biphenylyloxy- oder Naphthyloxygruppe, eine aliphatische, aromatische oder heterozyklische Acylgruppe oder ein Nitrogruppe bedeutet und die gestrichelte Linie bedeutet, daß eine Einfachbindung oder Doppelbindung in der gezeigten Stellung vorhanden ist, wobei, wenn die gestrichelte Linie eine Einfachbindung bedeutet, $R_3$ nicht Wasserstoff ist, und wenn die gestrichelte Linie eine Doppelbindung ist, $R_1$ ein Wasserstoffatom bedeutet und $R_4$ und $R_5$ beides Methylgruppen sind, $R_3$ nicht Wasserstoff ist) und pharmazeutisch verträgliche Salze derselben.

2. 2-(2,2-Dimethyl-8-fluor-1,2-benzopyran-6-yl)-propionsäure.

3. (2,2-Dimethyl-8-methoxy-1,2-benzopyran-6-yl)-essigsäure.

4. 2-(2,2-Dimethyl-8-chlorchroman-6-yl)-propionsäure.

5. Verfahren zur Herstellung einer Verbindung nach Anspruch 1 durch Umsetzung einer Verbindung der allgemeinen Formel (II):

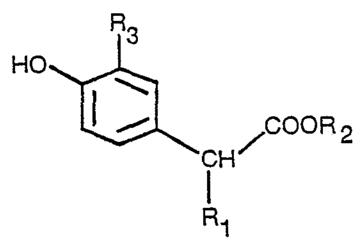

(II)

worin $R_1$, $R_2$ und $R_3$ wie in Anspruch 1 definiert sind, mit einer Verbindung der allgemeinen Formel

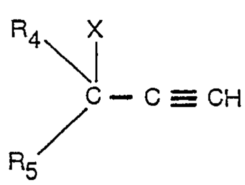

(III)

worin $R_4$ und $R_5$ wie in Anspruch 1 definiert sind und X ein Halogenatom ist, unter Bildung einer Verbindung der allgemeinen Formel (IV):

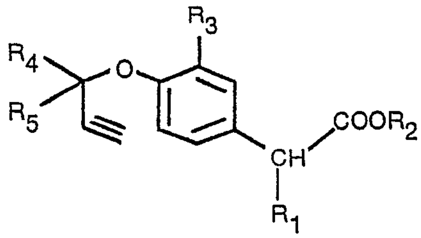

(IV)

worin $R_1$, $R_2$, $R_3$, $R_4$ und $R_5$ wie in Anspruch 1 definiert sind, Erwärmen der letzteren unter Ringschluß und gegebenenfalls Reduzieren, Hydrolysieren oder Umwandlung in ein Salz der resultierenden ringgeschlossenen Verbindung.

6. Verfahren nach Anspruch 5, bei dem die Verbindung der Formel (II) mit der Verbindung der Formel (III) in Gegenwart einer Base umgesetzt wird.

7. Verfahren nach einem der Ansprüche 5 und 6, bei dem der Ringschluß durch Erwärmen der Verbindung der Formel (IV) in einem Lösungsmittel bewirkt wird.

8. Pharmazeutisches Präparat mit wenigstens einer Verbindung nach einem der Ansprüche 1 bis 4 als aktiven Bestandteil zusammen mit einem pharmazeutischen Verdünnungsmittel, Träger oder Beschichtungsmittel.

9. Verbindung nach einem der Ansprüche 1 bis 4 für die Verwendung bei der Behandlung von Entzündungen, Fieber oder Schmerzen.

**Patentansprüche für den Vertragsstaat: AT**

1. Verfahren zur Herstellung von heterozyklischen Verbindungen der allgemeinen Formel (I):

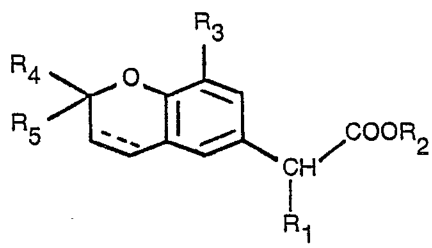

(I)

(worin jeweils $R_1$, $R_2$, $R_4$ und $R_5$ ein Wasserstoffatom oder eine $C_{1-6}$-Alkylgruppe bedeutet, $R_3$ ein Wasserstoffatom oder Halogenatom, eine $C_{1-8}$-Alkylgruppe, die gegebenenfalls durch eine $C_{3-6}$-Cycloalkylgruppe oder durch ein oder mehrere Halogenatome substituiert ist, eine $C_{1-8}$-Alkoxygruppe, eine $C_{2-7}$-Alkenylgruppe, eine $C_{2-7}$-Alkenyloxygruppe, eine Phenyl-, Tolyl-, Xylyl-, Biphenylyl-, Naphthyl-, Phenyloxy-, Tolyloxy-, Xylyloxy-, Biphenylyloxy- oder Naphthyloxygruppe, eine aliphatische, aromatische oder heterozyklische Acylgruppe oder ein Nitrogruppe bedeutet und die gestrichelte Linie bedeutet, daß eine Einfachbindung oder Doppelbindung in der gezeigten Stellung vorhanden ist, wobei, wenn die gestrichelte Linie eine Einfachbindung bedeutet, $R_3$ nicht Wasserstoff ist, und wenn die gestrichelte Linie eine Doppelbindung ist, $R_1$ ein Wasserstoffatom bedeutet und $R_4$ und $R_5$ beides Methylgruppen sind, $R_3$ nicht Wasserstoff ist) und pharmazeutisch verträglicher Salze derselben durch Umsetzung einer Verbindung der allgemeinen Formel (II):

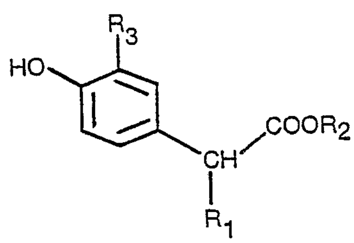

(II)

worin $R_1$, $R_2$ und $R_3$ wie in Anspruch 1 definiert sind, mit einer Verbindung der allgemeinen Formel (III):

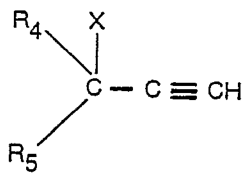

(III)

worin $R_4$ und $R_5$ wie in Anspruch 1 definiert sind und X ein Halogenatom bedeutet, unter Bildung einer Verbindung der allgemeinen Formel (IV):

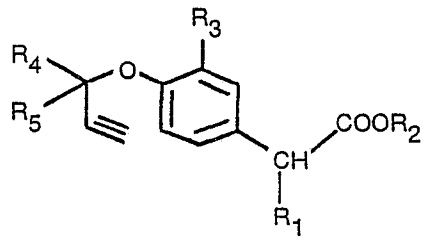

(IV)

worin $R_1$, $R_2$, $R_3$, $R_4$ und $R_5$ wie in Anspruch 1 definiert sind, Erhitzen der letzteren Verbindung unter Ringschluß und gegebenenfalls Reduzieren, Hydrolysieren oder Umwandlung in ein Salz der resultierenden ringgeschlossenen Verbindung.

2. Verfahren nach Anspruch 1, in dem die Verbindung der Formel (I) 2-(2,2-Dimethyl-8-fluor-1,2-benzopyran-6-yl)-propionsäure ist.

3. Verfahren nach Anspruch 1, in dem die Verbindung der Formel (I) (2,2-Dimethyl-8-methoxy-1,2-benzopyran-6-yl)-essigsäure ist.

4. Verfahren nach Anspruch 1, in dem die Verbindung der Formel (I) 2-(2,2-Dimethyl-8-chlorchroman-6-yl)-propionsäure ist.

5. Verfahren nach einem der vorausgehenden Ansprüche, bei dem die Verbindung der Formel (II) mit der Verbindung der Formel (III) in Gegenwart einer Base umgesetzt wird.

6. Verfahren nach einem der vorausgehenden Ansprüche, bei dem der Ringschluß durch Erwärmen der Verbindung der Formel (IV) in einem Lösungsmittel bewirkt wird.

7. Verfahren zur Herstellung eines pharmazeutischen Präparates durch Vereinigen einer Verbindung der Formel (I) gemäß einem der Ansprüche 1 bis 4 oder eines pharmazeutisch verträglichen Salzes derselben mit einem pharmazeutischen Verdünnungsmittel, Träger oder Beschichtungsmittel.

**Revendications pour les Etats contractants: BE CH DE FR GB IT LI LU NL SE**

1. Composés hétérocycliques de formule générale (I):

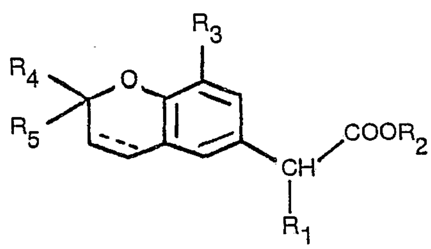

(I)

(dans laquelle chacun des radicaux $R_1$, $R_2$, $R_4$ et $R_5$ est un atome d'hydrogène ou un groupe alkyle en $C_{1-6}$; $R_3$ est un atome d'hydrogène ou d'halogène, un groupe alkyle en $C_{1-8}$ éventuellement substitué par un groupe cycloalkyle en $C_{3-6}$ ou par un ou plusieurs atomes d'halogène, un groupe alcoxy en $C_{1-8}$, un groupe alcényle en $C_{2-7}$, un groupe alcényloxy en $C_{2-7}$, un groupe phényle, tolyle, xylyle, biphénylyle, naphtyle, phényloxy, tolyloxy, xylyloxy, biphénylyloxy ou naphtyloxy, un groupe acyle aliphatique, aromatique ou hétérocyclique, ou un groupe nitro; et la ligne en tirets indique qu'une liaison simple ou double est présente dans la position indiquée; à la condition que, quand la ligne en tirets est une liaison simple, $R_3$ soit autre que l'hydrogène, et à la condition que, quand la ligne en tirets est une double liaison, que $R_1$ est un atome d'hydrogène et $R_4$ et $R_5$ tous les deux des groupes méthyle, $R_3$ soit autre que l'hydrogène) et leurs sels pharmaceutiquement acceptables.

2. Acide 2-(2,2-diméthyl-8-fluoro-1,2-benzopyranne-6-yl)-propionique.

3. Acide (2,2-diméthyl-8-méthoxy-1,2-benzopyranne-6-yl)-acétique.

4. Acide 2-(2,2-diméthyl-8-chlorochromanne-6-yl)-propionique.

5. Procédé pour la préparation d'un composé selon la revendication 1, qui consiste à faire réagir un composé de formule générale (II)

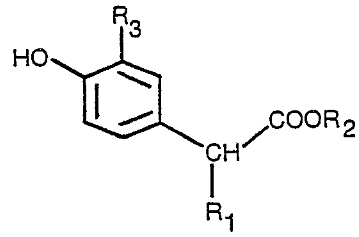

(II)

dans laquelle $R_1$, $R_2$ et $R_3$ sont comme défini dans la revendication 1, sur un composé de formule générale (III):

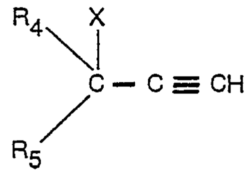

(III)

dans laquelle $R_4$ et $R_5$ sont comme défini dans la revendication 1 et X est un atome d'halogène, pour donner un composé de formule générale (IV):

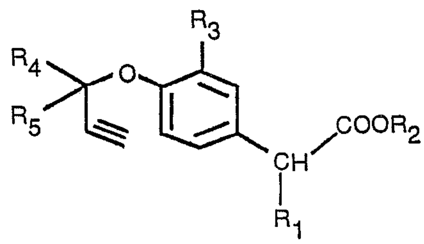

(IV)

dans laquelle $R_1$, $R_2$, $R_3$, $R_4$ et $R_5$ sont comme défini dans la revendication 1, à chauffer ce dernier pour réaliser la cyclisation et, si on le souhaite, à réduire le composé cyclisé obtenu, l'hydrolyser ou le convertir en un sel.

6. Procédé selon la revendication 5, dans laquel le composé de formule (II) réagit sur un composé de formule (III) en présence d'une base.

7. Procédé selon l'une quelconque des revendications 5 ou 6, dans lequel la cyclisation est réalisée par chauffage du composé de formule (IV) dans un solvant.

8. Composition pharmaceutique comprenant en tant que principe actif au moins un composé selon l'une quelconque des revendications 1 à 4, avec un diluant, excipient ou enrobage pharmaceutique.

9. Pour utilisation dans le traitement de l'inflammation, de la fièvre ou de la douleur, composé selon l'une quelconque des revendications 1 à 4.

**Revendications pour l'Etat contractant: AT**

1. Procédé pour la préparation de composés hétérocycliques de formule générale (I):

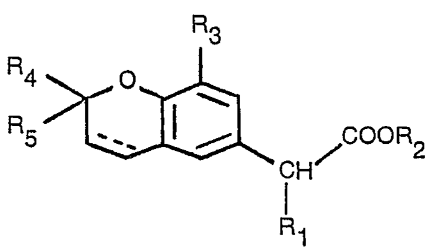

(I)

(dans laquelle chacun des radicaux $R_1$, $R_2$, $R_4$ et $R_5$ est un atome d'hydrogène ou un groupe alkyle en $C_{1-6}$; $R_3$ est un atome d'hydrogène ou d'halogène, un groupe alkyle en $C_{1-8}$ éventuellement substitué par un groupe cycloalkyle en $C_{3-6}$ ou par un ou plusieurs atomes d'halogène, un groupe alcoxy en $C_{1-8}$, un groupe alcényle en $C_{2-7}$, un groupe alcényloxy en $C_{2-7}$, un groupe phényle, tolyle, xylyle, biphénylyle, naphtyle, phényloxy, tolyloxy, xylyloxy, biphénylyloxy ou naphtyloxy, un groupe acyle aliphatique, aromatique ou hétérocyclique, ou un groupe nitro; et la ligne en tirets indique qu'une liaison simple ou double est présente dans la position indiquée; à la condition que, quand la ligne en tirets est une simple liaison, $R_3$ soit

autre que l'hydrogène, et à la condition que, quand la ligne en tirets est une double liaison, que $R_1$ est un atome d'hydrogène et $R_4$ et $R_5$ sont tous deux des groupes méthyle, $R_3$ soit autre que l'hydrogène) et leurs sels pharmaceutiquement acceptables, qui consiste à faire réagir un composé de formule générale (II):

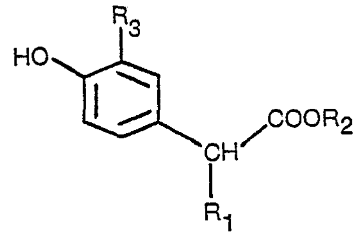

(II)

dans laquelle $R_1$, $R_2$ et $R_3$ sont comme défini dans la revendication 1, sur un composé de formule générale (III):

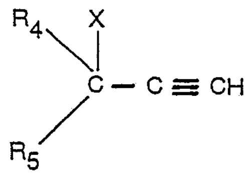

(III)

dans laquelle $R_4$ et $R_5$ sont comme défini dans la revendication 1 et X est un atome d'halogène, pour donner un composé de formule générale (IV):

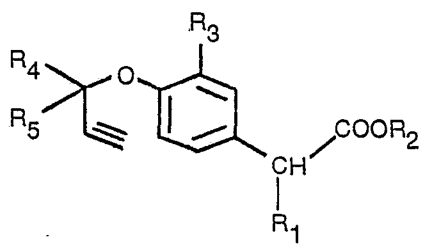

(IV)

dans laquelle $R_1$, $R_2$, $R_3$, $R_4$ et $R_5$ sont comme défini dans la revendication 1, à chauffer ce dernier pour réaliser la cyclisation et, si on le souhaite, à réduire le composé cyclisé obtenu, à l'hydrolyser ou le convertir en un sel.

2. Procédé selon la revendication 1, dans lequel le composé de formule (I) est l'acide 2-(2,2-diméthyl-8-fluoro-1,2-benzopyranne-6-yl)-propionique.

3. Procédé selon la revendication 1, dans lequel le composé de formule (I) est l'acide (2,2-diméthyl-8-méthoxy-1,2-benzopyranne-6-yl)-acétique.

4. Procédé selon la revendication 1, dans lequel le composé de formule (I) est l'acide 2-(2,2-diméthyl-8-chlorochromanne-6-yl)-propionique.

5. Procédé selon l'une quelconque des revendications précédentes, dans lequel le composé de formule (II) est mis à réagir sur le composé de formule (III) en présence d'une base.

6. Procédé selon l'une quelconque des revendications précédentes, dans lequel la cyclisation est réalisé par chauffage du composé de formule (IV) dans un solvant.

7. Procédé pour la préparation d'une composition pharmaceutique, qui consiste à combiner un composé de formule (I), tel que défini dans l'une quelconque des revendications 1 à 4, ou l'un de ses sels pharmaceutiquement acceptables, avec un diluant, excipient ou enrobage pharmaceutique.

FIG. 1.

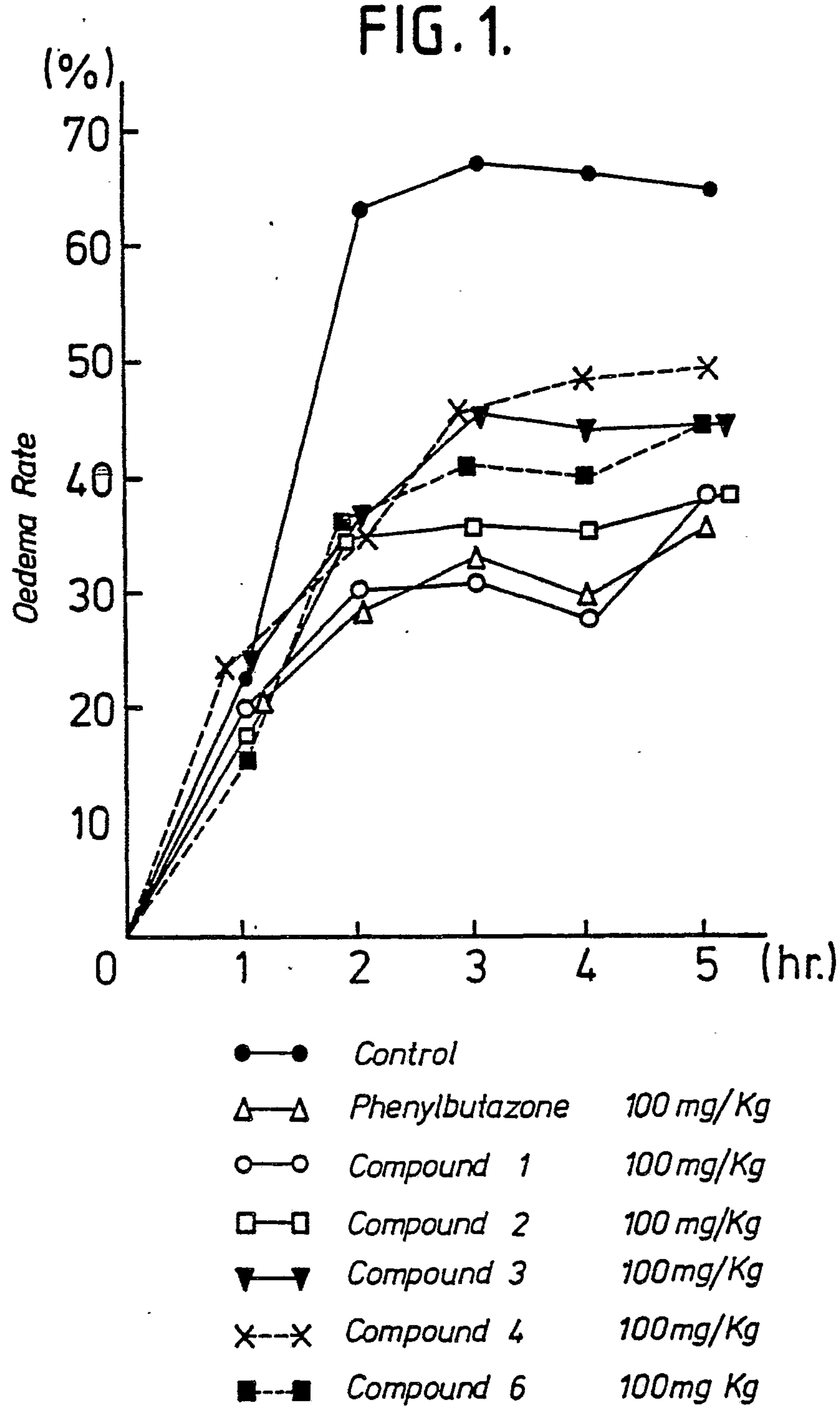
(%)
70
60
50
40
30
20
10
0
Oedema Rate
1
2
3
4
5
(hr.)
Control
Phenylbutazone 100 mg/Kg
Compound 1 100 mg/Kg
Compound 2 100 mg/Kg
Compound 3 100mg/Kg
Compound 4 100mg/Kg
Compound 6 100mg Kg

FIG. 2.

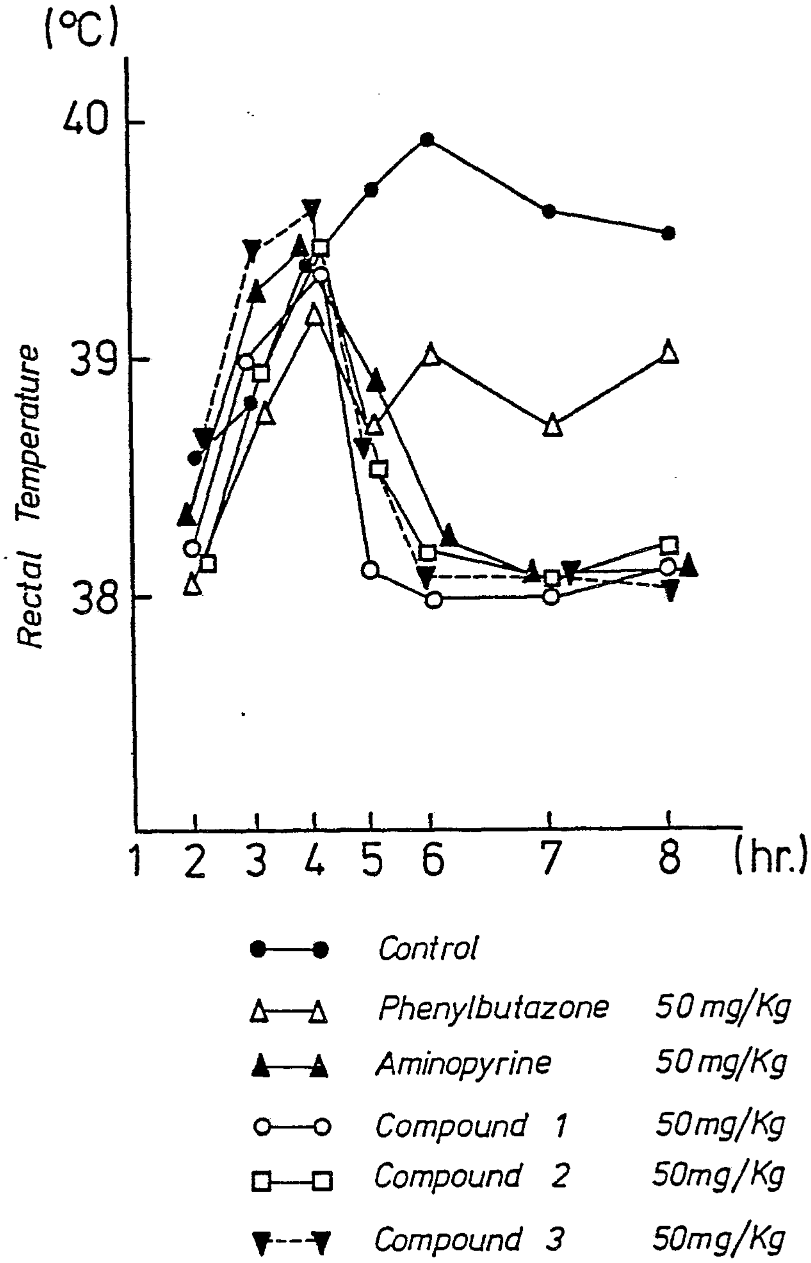
(°C)
40
39
38
Rectal Temperature
1
2
3
4
5
6
7
8
(hr.)
Control
Phenylbutazone 50 mg/Kg
Aminopyrine 50 mg/Kg
Compound 1 50mg/Kg
Compound 2 50mg/Kg
Compound 3 50mg/Kg